# EUROPEAN PATENT SPECIFICATION

(11) **EP 4 011 403 B1**
(45) Date of publication and mention of the grant of the patent: **28.05.2025**
(21) Application number: 21213040.5
(22) Date of filing: 08.12.2021
(51) Int. Cl.: A61L 2/10, A61L 9/20

(54) **PORTABLE SANITIZING SYSTEMS AND METHODS HAVING MODULAR COMPONENTS**
TRAGBARE DESINFEKTIONSSYSTEME UND VERFAHREN MIT MODULAREN KOMPONENTEN
SYSTÈMES ET PROCÉDÉS D'ASSAINISSEMENT PORTABLES AYANT DES COMPOSANTS MODULAIRES

(30) Priority: 10.12.2020 US 202063123517 P; 29.10.2021 US 202117514001
(43) Date of publication of application: 15.06.2022
(73) Proprietor: The Boeing Company, Arlington, VA 22202 (US)
(72) Inventor: CHILDRESS, Jamie J., CHICAGO, 60606-1596 (US); CALLAHAN, Kevin S., CHICAGO, 60606-1596 (US); BROWN, DOUGLAS ALAN, CHICAGO, 60606-1596 (US)
(74) Representative: Plasseraud IP

(56) References cited:
- CN-A- 108 904 833
- CN-A- 109 199 708
- CN-A- 111 388 100
- CN-U- 209 060 084
- CN-U- 211 048 664
- CN-U- 211 535 762
- DE-A1- 102017 107 210
- US-A1- 2014 264 084

## Description

### FIELD OF THE DISCLOSURE

Examples of the present disclosure generally relate to sanitizing systems, such as may be used to sanitize structures and areas within vehicles, such as commercial aircraft.

### BACKGROUND OF THE DISCLOSURE

Vehicles such as commercial aircraft are used to transport passengers between various locations. Systems are currently being developed to disinfect or otherwise sanitize surfaces within aircraft, in at least one example, that use ultraviolet (UV) light. In order to sanitize a surface of a structure, a known UV light sterilization method emits a broad spectrum UVC light onto the structure.

Further, known UV light sanitizing systems are typically large, bulky, and often require fixed, stationary infrastructure.

Additionally, UV light sanitizing systems include numerous components. If one of the components is in need of repair or replacement, the process of replacing the component is time and labor intensive. Moreover, when replacing or repairing the component, there is a potential of inadvertently damaging other components. As such, when a particular component of a system malfunctions, the entire system may be discarded and replaced, as the repair or replacement may exceed the cost of an entirely new system.

US 2014/264084 A1, in accordance with its abstract, states an ultraviolet sanitizer with flexible wand. A flexible wand is connected to a generator of ultraviolet radiation. The generator is mounted within a housing and connectable to a source of electrical energy. The distal wand end includes a switch for controlling the emission of the ultraviolet radiation from the wand with a distal tip directing the radiation toward the surface to be sanitized.

CN 108904833 A, in accordance with its abstract, states a portable medical equipment disinfection device. The portable medical equipment disinfection device includes a box body. The middle position of the bottom outer wall of the box body is connected with a first stirring motor through a fastening bolt, and the output shaft of the first stirring motor is connected with a stirring rod through a fastening bolt. The top of the circumference of the stirring rod is connected with first stirring paddles in equidistant distribution by fastening bolts. The top of the outer wall on one side of the box body is connected with a liquid storage tank by a fastening bolt.

CN 211048664 U, in accordance with its abstract, states a multifunctional convertible knapsack which comprises a knapsack body provided with straps, a main bag arranged on the knapsack body, a front bag arranged on the front side of the knapsack body, a feeding bottle bag and a lining bag. The feeding bottle bag is detachably arranged on the bag body; the outer side face of the lining bag is detachably connected with the inner side face of the main bag, and a locking structure used for sealing a bag opening of the lining bag is arranged on the lining bag.

CN 209060084 U, in accordance with its abstract, states a multifunctional intensive disinfection vehicle. The device comprises a shell, an ozone disinfection machine, a placing groove, a first electric push rod, a first ultraviolet sterilization lamp, a memory iron wire, a second ultraviolet sterilization lamp, a connecting pipeline, an external hose, a second electric push rod, an avoiding groove, a fixing plate and a garbage can.

DE 10-2017/107210 A1, in accordance with its abstract, states a handheld device for germ load reduction comprising a UV-C light source connected to a housing via a neck piece. A spacer attached to the neck piece that enables a defined irradiation distance to be maintained between the UV-C light source and the object to be treated.

CN 1113888100 A, in accordance with its abstract, states a portable nursing package for visiting a patient at home that comprises a package body that can be opened and closed, and at least one medical waste storage bag arranged on the outer side of the package body, wherein a plurality of matter storage compartments are arranged inside the package body, the top of each matter storage compartment is provide with an upward opening, ultraviolet lamps are installed on the inner side of the top of the package body, and the ultraviolet lamps are arranged to be turned on when the package body is closed.

CN 109199708 A, in accordance with its abstract, states a multifunctional nursing medical kit for emergency travel, including a rectangular box, wherein the left side of the rectangular box body is provided with a first storage tank, the first storage tank is slidably connected with the first honeycomb-shaped storage tank through a pulling guide provided at the lower end of the inner side thereof, the right end of the inner side of the first storage tank is provided with a semiconductor refrigerating sheet, the rectangular box is rotatably connected with a first sealing cover plate through a hinge arranged at the rear end of the left side surface thereof, the left and right sides of the inner center of the rectangular box body are respectively provided with a heat dissipation chamber and a battery chamber.

CN 211535762 U, in accordance with its abstract, states a nursing disinfection wherein the cover plate is arranged on one side of the shell, the fixing rod is in threaded connection with the bottom of an inner cavity of the shell, and the two partition plates are sequentially arranged on the outer side of the fixing rod in a sleeving mode. A through hole is formed in the fixed rod; a distribution control device for uniformly distributing the temperature among three interlayers formed by the two clapboards is arranged on the fixed rod.

### SUMMARY OF THE DISCLOSURE

A need exists for a UV sanitizing system and method in which component parts can be quickly and easily repaired or replaced, when needed. Further, a need exists for a UV sanitizing system that can be easily adapted for different needs.

With those needs in mind, certain examples of the present disclosure provide a portable sanitizing system according to claim 1.

As an example, the container may be a case assembly. As another example, the container may be a backpack assembly.

In at least one example, the one or more component modules may include a power supply, a battery pack, or a blower. Further, the one or more component modules can also include the wand assembly.

In at least one example, the container may also include one or more receptacles within the internal chamber. The one or more component modules may be configured to removably secure to the one or more receptacles through one or more coupling interfaces. As an example, the one or more coupling interfaces may include a plug and a socket, one or more latches, one or more detents, or one or more snaps.

In at least one example, the container may also include a radio frequency identification (RFID) tag.

In at least one example, the container may also include a global positioning system (GPS) device.

In at least one example, the system may also include a retaining frame secured to the container. The retaining frame is configured to house one or more additional component modules.

In at least one example, the container may also include a window that is configured to allow viewing into at least a portion of the internal chamber.

In at least one example, the one or more component modules may include a power supply. The power supply may include an activation switch and an information screen. Status indicators may be displayed on the information screen.

In at least one example, the system may further include a hose that connects the wand assembly to the container. The hose may include a first connector that removably connects to a second connector of the container.

Certain examples of the present disclosure, not forming part of the claimed subject matter, may provide a portable sanitizing method, including coupling a wand assembly to a container including an internal chamber. The wand assembly may include a sanitizing head having an ultraviolet (UV) lamp. The method may also include removably securing one or more component modules within the internal chamber.

### BRIEF DESCRIPTION OF THE DRAWINGS

Figure 1 illustrates a perspective view of a portable sanitizing system worn by an individual.
Figure 2 illustrates a perspective lateral top view of a wand assembly.
Figure 3 illustrates a perspective rear view of the wand assembly of Figure 2.
Figure 4 illustrates a perspective lateral view of the wand assembly of Figure 2.
Figure 5 illustrates a perspective view of the portable sanitizing system in a compact deployed position.
Figure 6 illustrates a perspective view of the portable sanitizing system having a sanitizing head in an extended position.
Figure 7 illustrates a perspective view of the portable sanitizing system having the sanitizing head in an extended position and a handle in an extended position.
Figure 8 illustrates a perspective view of the portable sanitizing system having the sanitizing head rotated in relation to the handle.
Figure 9 illustrates a perspective end view of a UV lamp and a reflector of the sanitizing head.
Figure 10 illustrates a perspective end view of a UV lamp and a reflector of the sanitizing head.
Figure 11 illustrates a perspective end view of a UV lamp and a reflector of the sanitizing head.
Figure 12 illustrates a perspective top view of the sanitizing head.
Figure 13 illustrates a perspective bottom view of the sanitizing head.
Figure 14 illustrates an axial cross-sectional view of the sanitizing head through line 14-14 of Figure 12.
Figure 15 illustrates a perspective end view of the UV lamp secured to a mounting bracket.
Figure 16 illustrates a perspective exploded view of a backpack assembly, according to an example of the present disclosure.
Figure 17 illustrates a perspective front view of a harness coupled to a backpack assembly.
Figure 18 illustrates an ultraviolet light spectrum.
Figure 19 illustrates a perspective view of a portable sanitizing system.
Figure 20 illustrates a perspective view of the portable sanitizing system having a case assembly in an open position.
Figure 21 illustrates a perspective view of the portable sanitizing system having the case assembly in the open position.
Figure 22 illustrates a perspective view of the portable sanitizing system having the case assembly in the open position.
Figure 23 illustrates a perspective lateral view of the wand assembly.
Figure 24 illustrates a perspective bottom view of the wand assembly of Figure 23.
Figure 25 illustrates a perspective bottom view of the wand assembly of Figures 23 and 24 without the UV lamp.
Figure 26 illustrates a perspective view of a cooling manifold of a shroud of the wand assembly.
Figure 27 illustrates a schematic block diagram of a portable sanitizing system.
Figure 28 illustrates a simplified view of a coupling interface between a component module and a receptacle.
Figure 29 illustrates a simplified view of a coupling interface between a component module and a receptacle.
Figure 30 illustrates a simplified view of a coupling interface between a component module and a receptacle.
Figure 31 illustrates a simplified view of a coupling interface between a component module and a receptacle.
Figure 32 illustrates a perspective view of a portable sanitizing system.
Figure 33 illustrates a top view of the portable sanitizing system of Figure 32.
Figure 34 illustrates a perspective view of a portable sanitizing system.
Figure 35 illustrates a top view of the portable sanitizing system of Figure 34.
Figure 36 illustrates a perspective side of a portable sanitizing system.
Figure 37 illustrates a perspective top view of a portable sanitizing system.
Figure 38 illustrates a perspective lateral view of a portable sanitizing system.
Figure 39 illustrates a simplified view of a coupling interface between a hose and a container.
Figure 40 illustrates a perspective end view of a hose.
Figure 41 illustrates a perspective top view of a wire retainer.
Figure 42 illustrates a perspective view of electrical wires retained by the wire retainer.
Figure 43 illustrates a perspective front view of an aircraft.
Figure 44A illustrates a top plan view of an internal cabin of an aircraft.
Figure 44B illustrates a top plan view of an internal cabin of an aircraft.
Figure 45 illustrates a perspective interior view of an internal cabin of an aircraft.
Figure 46 illustrates a perspective internal view of a lavatory within an internal cabin of an aircraft.
Figure 47 illustrates a flow chart of a portable sanitizing method.

### DETAILED DESCRIPTION OF THE DISCLOSURE

The foregoing summary, as well as the following detailed description of certain examples will be better understood when read in conjunction with the appended drawings. As used herein, an element or step recited in the singular and preceded by the word "a" or "an" should be understood as not necessarily excluding the plural of the elements or steps. Further, references to "one example" are not intended to be interpreted as excluding the existence of additional examples that also incorporate the recited features. Moreover, unless explicitly stated to the contrary, examples "comprising" or "having" an element or a plurality of elements having a particular condition can include additional elements not having that condition.

Certain examples of the present disclosure provide a sanitizing system that includes a container (such as a case assembly, a backpack assembly, a cart assembly, or the like) that contains a plurality of component modules. Examples of the component modules include a battery pack, a power supply, a blower fan, a wand assembly with a hose attachment, and the like. The component modules are configured to be removably secured within the container. As such, the component modules can be quickly and easily removed, repaired outside of the container, and/or replaced with another component module. Further, the component modules can be removed from a first container, such as of a case assembly, a backpack assembly, or a cart assembly, and inserted into a second container that differs from the first container, such as another one of a case assembly, a backpack assembly, or a cart assembly. Examples of the present disclosure provide modularity and ease of repair of portions of the UV sanitizing system.

In at least one example, the UV wand assembly and/or a hose assembly can be quickly and easily unplugged from a power module, and plugged into an alternative power module without the use of tools. The UV wand assembly and hose assembly can be plugged into a power module of a backpack assembly, a roller bag. A shoulder bag, a fixed location, and/or the like.

In at least one example, the UV sanitizing system also includes a microcontroller that is configured to detect component status and compatibility. The microcontroller is configured to adjust system parameters based on configuration. The microcontroller can display a status light and optional informational display message to provide status information, such as fault conditions, diagnostic data, and battery state. In at least one example, the microcontroller is configured to detect the make and model of attached accessories, based on a connector pin arrangement. The microcontroller compares the installed pin arrangement data to known arrangements to determine compatibility of the accessory, and may adjust the system parameters to optimize the system.

In at least one example, the hose connecting the UV wand to the system may be attached to the system with a threaded retainer, and may contain a wire strain relief retainer inside the hose to prevent high voltage wires from detaching during the removal and replacement process.

Examples of the present disclosure provide plug and play modularity for components of a UV sanitizing system to allow for easier removal and replacement of components. The component modules can be inserted and removed from a container (such as a backpack assembly, case assembly, cart, or the like) without the use of tools..

In at least one example, the UV sanitizing system can be tracked and monitored via a global positioning system (GPS)-enabled asset management system. The asset management system can provide geographic location wirelessly to a control center and/or one or more handheld devices, such as smart phones, smart tablets, laptop computers, or other computing devices.

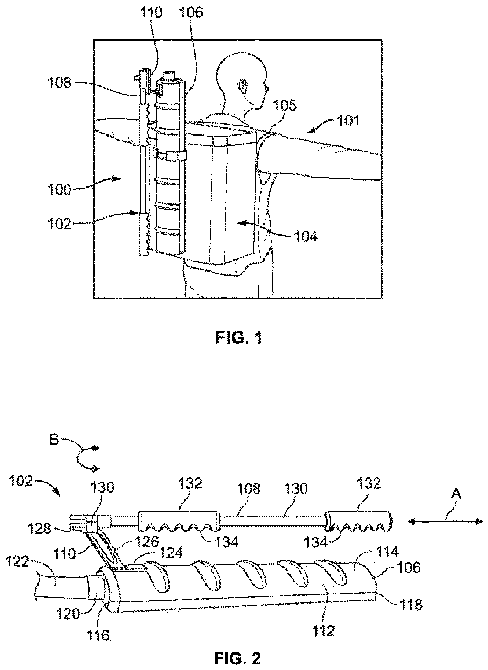

Figure 1 illustrates a perspective view of a portable sanitizing system 100 worn by an individual 101, according to an example of the present disclosure. The portable sanitizing system 100 includes a wand assembly 102 coupled to a backpack assembly 104 that is removably secured to the individual through a harness 105. The wand assembly 102 includes a sanitizing head 106 coupled to a handle 108. In at least one example, the sanitizing head 106 is moveably coupled to the handle 108 through a coupler 110.

In at least one other example, the portable sanitizing system 100 may not be worn by the individual 101. For example, the portable sanitizing system 100 may include a case assembly that is configured to be opened and closed. The case assembly may store the wand assembly 102 when not in use. The case assembly may be opened to allow the wand assembly 102 to be removed and operated.

As shown in Figure 1, the wand assembly 102 is in a stowed position. In the stowed position, the wand assembly 102 is removably secured to a portion of the backpack assembly 104, such as through one or more tracks, clips, latches, belts, ties, and/or the like.

In at least one other example, the wand assembly 102 is stored within a case assembly in a stowed position. For example, the wand assembly 102 in the stowed position is contained within a closed case assembly. The case assembly may be opened to allow the wand assembly 102 to be removed and deployed.

Figure 2 illustrates a perspective lateral top view of the wand assembly 102, according to an example of the present disclosure. The sanitizing head 106 couples to the handle 108 through the coupler 110. The sanitizing head 106 includes a shroud 112 having an outer cover 114 that extends from a proximal end 116 to a distal end 118. As described herein, the shroud 112 contains a UV lamp.

Optionally, the wand assembly 102 may include the sanitizing head 106 connected to a fixed handle. Further, the wand assembly 102 may be sized and shaped differently than shown.

A port 120 extends from the proximal end 116. The port 120 couples to a hose 122, which, in turn, couples to the backpack assembly 104 (shown in Figure 1). The hose 122 contains electrical cords, cables, wiring, or the like that couples a power source or supply (such as one or more batteries) within the backpack assembly 104 (shown in Figure 1) to a UV lamp 140 within the shroud 112. Optionally, the electrical cords, cables, wiring, or the like may be outside of the hose 122. In at least one example, the hose 122 also contains an air delivery line, such as an air tube) that fluidly couples an internal chamber of the shroud 112 to an air blower, vacuum generator, air filters, and/or the like within the backpack assembly 104.

The coupler 110 is secured to the outer cover 114 of the shroud 112, such as proximate to the proximal end 116. The coupler 110 may include a securing beam 124 secured to the outer cover 114, such as through one or more fasteners, adhesives, and/or the like. An extension beam 126 outwardly extends from the securing beam 124, thereby spacing the handle 108 from the shroud 112. A bearing assembly 128 extends from the extension beam 126 opposite from the securing beam 124. The bearing assembly 128 includes one or more bearings, tracks, and/or the like, which allow the handle 108 to linearly translate relative to the coupler 110 in the directions of arrows A, and/or pivot about a pivot axle in the directions of arc B. Optionally, the securing beam 124 may include a bearing assembly that allows the sanitizing head 106 to translate in the directions of arrows A, and/or rotate (in at least one example, swivel) in the directions of arc B in addition to, or in place of, the handle 108 being coupled to the bearing assembly 128 (in at least one example, the handle 108 may be fixed to the coupler 110).

In at least one other example, the wand assembly 102 does not include the coupler 110. Instead, the handle 108 may be fixed to the shroud 112, in at least one example.

In at least one example, the handle 108 includes a rod, pole, beam, or the like 130, which may be longer than the shroud 112. Optionally, the rod 130 may be shorter than the shroud 112. One or more grips 132 are secured to the rod 130. The grips 132 are configured to be grasped and held by an individual. The grips 132 may include ergonomic tactile features 134.

Optionally, the wand assembly 102 can be sized and shaped differently than shown. For example, in at least one example, the handle 108 can be fixed in relation to the shroud 112. Further, the handle 108 may not be configured to move relative to itself and/or the shroud 112. For example, the handle 108 and the shroud 112 can be integrally molded and formed as a single unit.

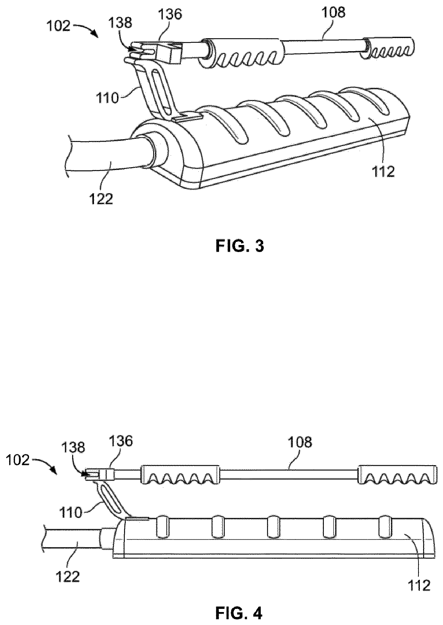

Figure 3 illustrates a perspective rear view of the wand assembly 102 of Figure 2. Figure 4 illustrates a perspective lateral view of the wand assembly 102 of Figure 2. Referring to Figures 3 and 4, the handle 108 may pivotally couple to the coupler 110 through a bearing 136 having a pivot axle 138 that pivotally couples the handle 108 to the coupler 110. The handle 108 may further be configured to linearly translate into and out of the bearing 136. For example, the handle 108 may be configured to telescope in and out. Optionally, or alternatively, in at least one example, the handle 108 may include a telescoping body that allows the handle 108 to outwardly extend and inwardly recede. In at least one other example, the handle 108 may not be configured to move, extend, retract, or the like relative to the shroud 112.

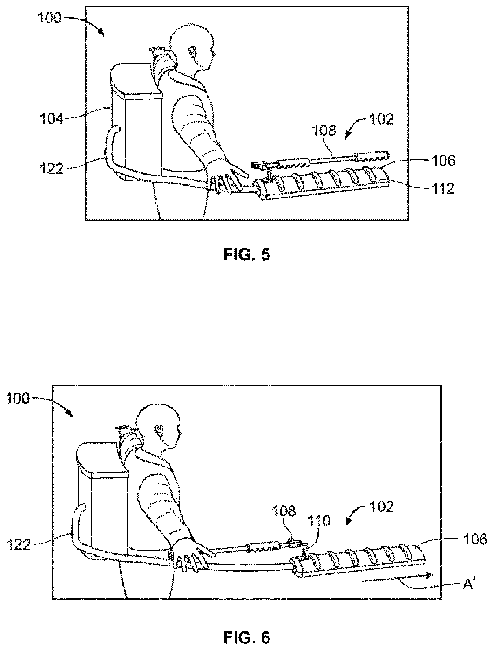

Figure 5 illustrates a perspective view of the portable sanitizing system 100 in a compact deployed position, according to an example of the present disclosure. The wand assembly 102 is removed from the backpack assembly 104 (as shown in Figure 1) into the compact deployed position, as shown in Figure 5. The hose 122 connects the wand assembly 102 to the backpack assembly 104. In the compact deployed position, the sanitizing head 106 is fully retracted in relation to the handle 108.

Figure 6 illustrates a perspective view of the portable sanitizing system 100 having the sanitizing head 106 in an extended position, according to an example of the present disclosure. In order to extend the sanitizing head 106 relative to the handle 108, the sanitizing head 106 is outwardly slid relative to the handle 108 in the direction of arrow A' (or the handle 108 is rearwardly slid relative to the sanitizing head 106). As noted, the sanitizing head 106 is able to linearly translate in the direction of arrow A' relative to the handle 108 via the coupler 110. The outward extension of the sanitizing head 106, as shown in Figure 6, allows for the portable sanitizing system 100 to easily reach distant areas. Alternatively, the sanitizing head 106 may not linearly translate relative to the handle 108.

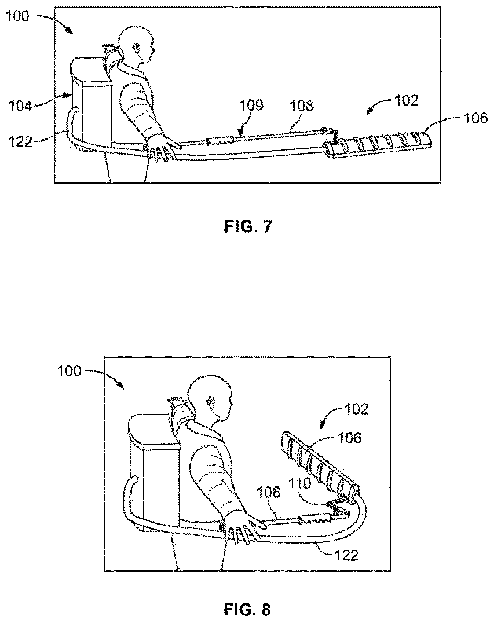

Figure 7 illustrates a perspective view of the portable sanitizing system 100 having the sanitizing head 106 in an extended position and the handle 108 in an extended position, according to an example of the present disclosure. To reach even further, the handle 108 may be configured to linearly translate, such as through a telescoping portion, to allow the sanitizing head 106 to reach further outwardly. Alternatively, the handle 108 may not be configured to extend and retract.

In at least one example, the handle 108 may include a lock 109. The lock 109 is configured to be selectively operated to secure the handle 108 into a desired extended (or retracted) position.

Figure 8 illustrates a perspective view of the portable sanitizing system 100 having the sanitizing head 106 rotated in relation to the handle 108, according to an example of the present disclosure. As noted, the sanitizing head 106 is configured to rotate relative to the handle 108 via the coupler 110. Rotating the sanitizing head 106 relative to the handle 108 allows the sanitizing head 106 to be moved to a desired position, and sweep or otherwise reach into areas that would otherwise be difficult to reach if the sanitizing head 106 was rigidly fixed to the handle 108. Alternatively, the sanitizing head 106 may not be rotatable relative to the handle 108.

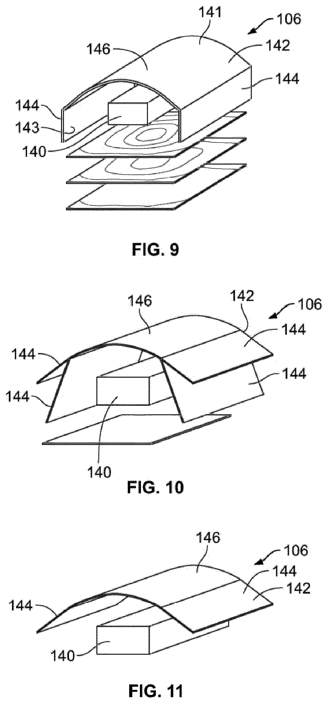

Figure 9 illustrates a perspective end view of a UV lamp 140 and a reflector 142 of the sanitizing head 106, according to an example of the present disclosure. The UV lamp 140 and the reflector 142 are secured within the shroud 112 (shown in Figure 2, in at least one example) of the sanitizing head 106. In at least one example, the reflector 142 is secured to an underside 141 of the shroud 112, such as through one or more adhesives. As another example, the reflector 142 is an integral part of the shroud 112. For example, the reflector 142 may be or otherwise provide the underside 141 of the shroud 112. The reflector 142 provides a reflective surface 143 (such as formed of Teflon, a mirrored surface, and/or the like) that is configured to outwardly reflect UV light emitted by the UV lamp 140. In at least one example, shroud 112 may be or include a shell formed of fiberglass, and the reflector 142 may be formed of Teflon that provides a 98% reflectivity. In at least one example, the reflector 142 may be a multi-piece reflector.

The reflector 142 may extend along an entire length of the underside 141 of the shroud 112. Optionally, the reflector 142 may extend along less than an entire length of the underside 141 of the shroud 112.

The UV lamp 140 may extend along an entire length (or along substantially the entire length, such as between the ends 116 and 118). The UV lamp 140 is secured to the reflector 142 and/or the shroud 112 through one or more mounts, such as brackets, in at least one example. The UV lamp 140 includes one or more UV light emitters, such as one more bulbs, light emitting elements (such as light emitting diodes), and/or the like. In at least one example, the UV lamp 140 is configured to emit UV light in the far UV spectrum, such as at a wavelength between 200 nm - 230 nm. In at least one example, the UV lamp 140 is configured to emit UV light having a wavelength of 222 nm. For example, the UV lamp 140 may be or include a 300 W bulb that is configured to emit UV light having a wavelength of 222 nm. Alternatively, the UV lamp 140 may be configured to emit UV light in other portions of the UV spectrum, such as the UVC spectrum. In at least one example, the UV lamp 140 is configured to emit UV light having a wavelength of 254 nm.

As shown, the reflector 142 includes flat, upright side walls 144 connected together through an upper curved wall 146. The upper curved wall 146 may be bowed outwardly away from the UV lamp 140. For example, the upper curved wall 146 may have a parabolic cross-section and/or profile.

It has been found that the straight, linear side walls 144 provide desired reflection and/or focusing of UV light emitted from the UV lamp 140 toward and onto a desired location. Alternatively, the side walls 144 may not be linear and flat.

Figure 10 illustrates a perspective end view of the UV lamp 140 and a reflector 142 of the sanitizing head, according to an example of the present disclosure. The reflector 142 shown in Figure 10 is similar to the reflector 142 shown in Figure 9, except that the side walls 144 may outwardly cant from the upper curved wall 146.

Figure 11 illustrates a perspective end view of the UV lamp 140 and the reflector 142 of the sanitizing head, according to an example of the present disclosure. In this example, the side walls 144 may be curved according to the curvature of the upper curved wall 146.

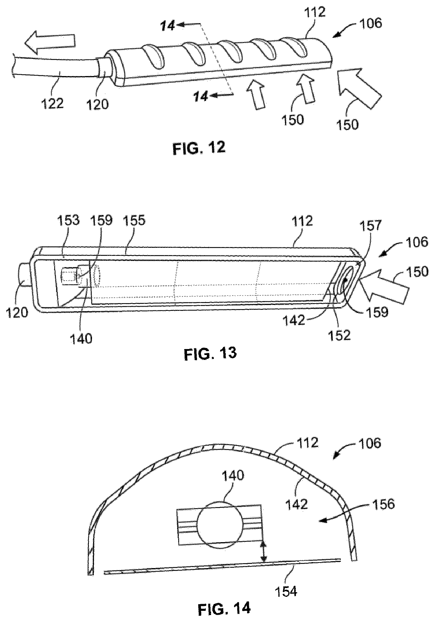

Figure 12 illustrates a perspective top view of the sanitizing head 106. Figure 13 illustrates a perspective bottom view of the sanitizing head 106. Figure 14 illustrates an axial cross-sectional view of the sanitizing head 106 through line 14-14 of Figure 12. Referring to Figures 12-14, air 150 is configured to be drawn into the sanitizing head 106 through one or more openings 152 (or simply an open chamber) of the shroud 112. The air 150 is drawn into the sanitizing head 106, such as via a vacuum generator within the backpack assembly 104 (shown in Figure 1). The air 150 is drawn into the shroud 112, and cools the UV lamp 140 as it passes over and around the UV lamp 140. The air 150 passes into the port 120 and into the hose 122, such as within an air tube within the hose 122. The air 150 not only cools the UV lamp 140, but also removes ozone, which may be generated by operation of the UV lamp 140, within the shroud 112. The air 150 may be drawn to an air filter, such as an activated carbon filter, within the backpack assembly 104.

In at least one example, the portable sanitizing system 100 may also include an alternative ozone mitigation system. As an example, the ozone mitigation system may be disposed in the shroud 112 or another portion of the system, and may include an inert gas bath, or a face inert gas system, such as in U.S. Patent No. 10,232,954.

Referring to Figure 13, in particular, a bumper 153 may be secured to an exposed lower circumferential edge 155 of the shroud 112. The bumper 153 may be formed of a resilient material, such as rubber, another elastomeric material, open or closed cell foam, and/or the like. The bumper 153 protects the sanitizing head 106 from damage in case the sanitizing head 106 inadvertently contacts a surface. The bumper 153 also protects the surface from damage.

The openings 152 may be spaced around the lower surface of the shroud 112 such that they do not provide a direct view of the UV lamp 140. For example, the openings 152 may be positioned underneath portions that are spaced apart from the UV lamp 140.

Referring to Figure 14, in particular, the sanitizing head 106 may include a cover plate 154 below the UV lamp 140. The cover plate 154 may be formed of glass, in at least one example, and may be configured to filter UV light emitted by the UV lamp 140. The UV lamp 140 may be secured within an interior chamber 156 defined between the reflector 142 and the cover plate 154. In at least one example, the cover plate 154 is or otherwise includes a far UV band pass filter. For example, the cover plate 154 may be a 222 nm band pass filter that filters UV light emitted by the UV lamp 140 to a 222 nm wavelength. As such, UV light that is emitted from the sanitizing head 106 may be emitted at a wavelength of 222 nm.

Referring to Figures 13 and 14, a rim 157 (such as a 0.020" thick Titanium rim) may connect the cover plate 154 to the shroud 112. The rim 157 may distribute impact loads therethrough and/or therearound.

In at least one example, ranging light emitting diodes (LEDs) 159 may be disposed proximate to ends of the UV lamp 140. The ranging LEDs 159 may be used to determine a desired range to a structure that is to be sanitized, in at least one example. In at least one example, the ranging LEDs 159 may be disposed on or within the rim 157 and/or the cover plate 154. As another example, the sanitizing head 106 may be configured for range guidance, as disclosed in United States Provisional Application No. 63/027,869, which was filed May 20, 2020.

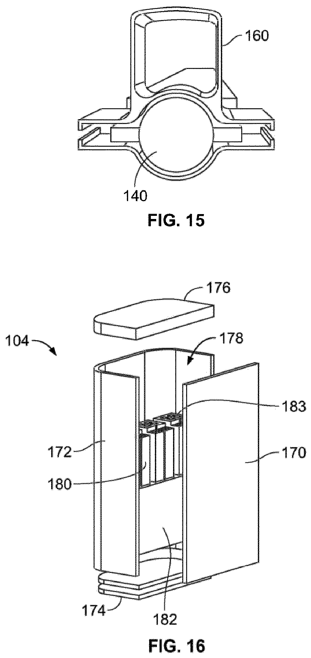

Figure 15 illustrates a perspective end view of the UV lamp 140 secured to a mounting bracket or clamp 160, according to an example of the present disclosure. Each end of the UV lamp 140 may be coupled to mounting bracket or clamp 160, which secures the UV lamp 140 to the shroud 112 (shown in Figures 12-14). A buffer, such as a thin (in at least one example, 0.040") sheet of silicon may be disposed between the end of the UV lamp 140 and the bracket 160. Optionally, the UV lamp 140 may be secured to the shroud 112 through brackets or clamps that differ in size and shape than shown. As another example, the UV lamp 140 may be secured to the shroud 112 through adhesives, fasteners, and/or the like.

Figure 16 illustrates a perspective exploded view of the backpack assembly 104, according to an example of the present disclosure. The backpack assembly 104 includes a front wall 170 that couples to a rear shell 172, a base 174, and a top cap 176. An internal chamber 178 is defined between the front wall 170, the rear shell 172, the base 174, and the top cap 176. One or more batteries 180, such as rechargeable Lithium batteries, are contained within the internal chamber 178. An air generation sub-system 182 is also contained within the internal chamber 178. The air generation sub-system 182 is in fluid communication with an air tube within the hose 122 (shown in Figure 2, in at least one example). The air generation sub-system 182 may include an airflow device, such as a vacuum generator, an air blower, and/or the like. The airflow device is configured to generate airflow to cool the UV lamp, draw air from the sanitizing head 106 into the backpack assembly 104 and out through an exhaust, draw or otherwise remove generated ozone away from the shroud 112, and/or the like.

One or more air filters 183, such as carbon filters, are within the backpack assembly 104. The air filters 183 are in communication with the air tube or other such delivery duct or line that routes air through the hose 122 and into the backpack assembly 104. The air filters 183 are configured to filter the air that is drawn into the backpack assembly 104 from the shroud 112. For example, the air filters 183 may be configured to remove, deactivate, or otherwise neutralize ozone.

The batteries 180 and/or a power supply within the backpack assembly 104 provides operating power for the UV lamp 140 of the sanitizing head 106 (shown in Figure 2, in at least one example). The top wall 176 may be removably coupled to the front wall 170 and the rear shell 172. The top wall 176 may be removed to provide access to the batteries 180 (such as to remove and/or recharge the batteries), in at least one example. Additional space may be provided within the backpack assembly 104 for storage of supplies, additional batteries, additional components, and/or the like. In at least one example, the front wall 170, the rear shell 172, the base 174, and the top cap 176 may be formed of fiberglass epoxy.

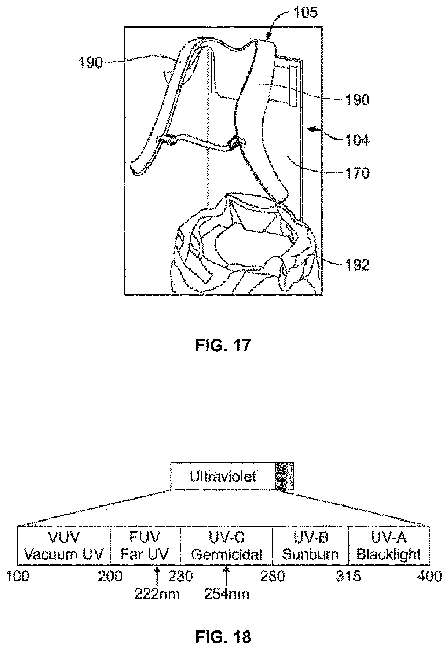

Figure 17 illustrates a perspective front view of the harness 105 coupled to the backpack assembly 104, according to an example of the present disclosure. The harness 105 may include shoulder straps 190 and/or a waist or hip belt or strap 192, which allow the individual to comfortably wear the backpack assembly 104.

Referring to Figures 1-17, in operation, the individual may walk through an area wearing the backpack assembly 104. When a structure to be sanitized is found, the individual may position grasp the handle 108 and position the sanitizing head 106 as desired, such as by extending and/or rotating the sanitizing head 106 relative to the handle 108. The individual may then engage an activation button on the handle 108, in at least one example, to activate the UV lamp 140 to emit sanitizing UV light onto the structure. As the UV lamp 140 is activated, air 150 is drawn into the shroud 112 to cool the UV lamp 140, and divert any generated ozone into the backpack assembly 104, where it is filtered by the air filters 183.

The extendable wand assembly 102 allows the sanitizing head 106 to reach distant areas, such as over an entire set of three passenger seats, from a row within an internal cabin of a commercial aircraft.

Figure 18 illustrates an ultraviolet light spectrum. Referring to Figures 1-18, in at least one example, the sanitizing head 106 is configured to emit sanitizing UV light (through operation of the UV lamp 140) within a far UV spectrum, such as between 200 nm to 230 nm. In at least one example, the sanitizing head 106 emits sanitizing UV light having a wavelength of 222 nm.

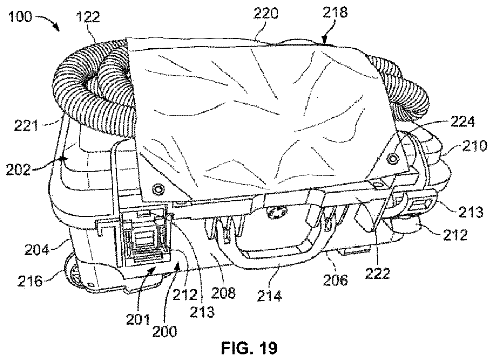

Figure 19 illustrates a perspective view of a portable sanitizing system 100, according to an example of the present disclosure. The portable sanitizing system 100 includes a case assembly 200 that is configured to store the wand assembly 102 (hidden from view in Figure 19) when the case assembly 200 is in a closed position, as shown in Figure 19.

The case assembly 200 may be formed of plastic, in at least one example. The case assembly 200 includes a main body 201, such as a shell, lower body portion, or the like. A cover 202, such as a lid, or upper body portion, is moveably coupled to the main body 201. For example, the cover 202 may be coupled to the main body 201 through a hinge that allows the cover 202 to be opened and closed relative to the main body 201.

The main body 201 includes a base 204 connected to a rear wall 206, lateral walls 208, and a top wall 210. The cover 202 is moveably coupled to a first lateral wall 208, such as through a hinge. One or more latches 212 are disposed on a second lateral wall 208, opposite from the first lateral wall 208. The latches 212 are configured to engage one or more reciprocal latch members 213 extending from the cover 202 to secure the cover 202 in the closed position. The latches 212 may be engaged by an individual to disengage the latch members 213 to allow the cover 202 to be pivoted into an open position.

A handle 214 is secured to the case assembly 200. For example, the handle 214 is pivotally secured to a lateral wall 208. The handle 214 is configured to be grasped by an individual so that the portable sanitizing system 100 may be carried. Optionally, the handle 214 may be secured to other portions of the case assembly 200, such as the top wall 210. In at least one example, the handle 214 may be configured to retract into the case assembly 200 into a fully retracted position, and extend out of (in at least one example, telescope out of) the case assembly 200 into a fully extended position.

Casters 216 or other such wheels may be rotatably secured to a portion of the case assembly 200. For example, two casters 216 may be rotatably secured to the base 204 proximate to the rear wall 206. An individual may tilt the case assembly 200 so that the casters 216 contact a floor. In this manner, the individual may roll the portable sanitizing system 100 via the casters 216 (and optionally through a handle in an extended position from the top wall 210). Alternatively, the case assembly 200 may not include the casters 216.

The hose 122 may outwardly extend from the case assembly 200. In the closed position, when the wand assembly 102 is in a stowed position within the case assembly 200, the hose 122 may be coiled over the cover 202. A hose retainer 218 may secure the hose 122 in place on the cover 202. For example, the hose retainer 218 may include a flexible fabric sheet 220 that is secured to a first side 221 of the cover 202, and may removably secured to an opposite second side 222 of the cover 202, such as through one or more fastening members 224, such as hooks and loops, latches, clips, and/or the like. The hose retainer 218 is configured to secure the hose 122 on the cover 202 when the wand assembly 102 is within a storage chamber of the case assembly 200 and the cover 202 is in a closed position. Alternatively, the hose 122 may be contained within a storage chamber of the case assembly 200 when the wand assembly 102 is not in use. That is, the storage chamber may be sized and shaped to also contain the hose 122 when the wand assembly 102 is also within the storage chamber and the cover 202 is in the closed position.

The wand assembly 102 within the case assembly 200 in the closed position is protected from inadvertent engagement, bumping, and the like. That is, by storing the wand assembly 102 within the case assembly 200, which is closed, when the wand assembly 102 is not in use, the portable sanitizing system 100 protects the wand assembly 102 from potential damage, and increases the useful life of the wand assembly 102.

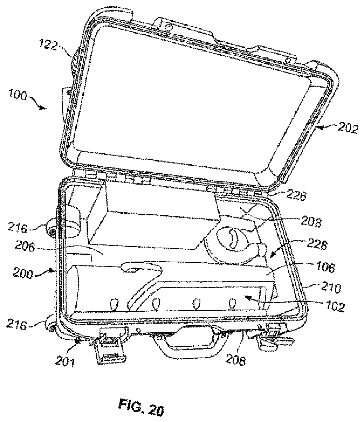

Figure 20 illustrates a perspective view of the portable sanitizing system 100 having the case assembly 200 in an open position, according to an example of the present disclosure. As shown, the cover 202 is opened via a hinge 226 that pivotally couples the cover 202 to the main body 201.

An internal or storage chamber 228 is defined between the base 204, the lateral walls 208, the rear wall 206, and the top wall 210 (and the cover 202, when closed). Various components of the portable sanitizing system 100 may be stored within the storage chamber 228. For example, the components within the backpack assembly 104, as described with respect to Figure 16, may be contained within the storage chamber 228.

For example, when not in use, the wand assembly 102 is contained within the storage chamber 228. Additionally, one or more batteries, such as rechargeable Lithium batteries, may be contained within the storage chamber 228.

An air generation sub-system (such as a cooling fan) may also be contained within the storage chamber 228. The air generation sub-system may be in fluid communication with an air tube within the hose 122. The hose 122 may be removably connected to the air generation sub-system. In at least one example, the hose 122 is configured to be coupled to and uncoupled from the wand assembly 102 and the air generation sub-system. That is, the hose 122 may be removably coupled to the wand assembly 102 and the air generation sub-system.

One or more air filters, such as carbon filters, may also be within the storage chamber 228. The air filters may be in communication with the air tube or other such delivery duct or line that routes air through the hose 122.

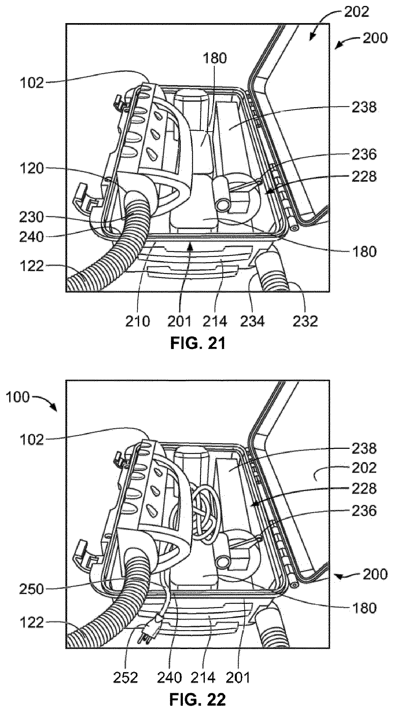

Figure 21 illustrates a perspective view of the portable sanitizing system 100 having the case assembly 200 in the open position, according to an example of the present disclosure. The wand assembly 102 is configured to be stowed in the storage chamber 228. When the wand assembly 102 is to be used, the cover 202 is opened, and a first end 230 of the hose 122 is coupled to the port 120 of the wand assembly 102. In at least one example, the hose 122 is configured to channel cooling air into the wand assembly 102, in order to cool the UV lamp 140 during activation.

A second end 232 of the hose 122 may be connected to a port 234 extending into and through a portion of the main body 201, such as through a portion of the top wall 210. The port 234 connects the hose 122 to an air generation sub-system, such as a cooling fan 236 that is within the storage chamber 228. The cooling fan 236 may be activated to generate cooling air that is delivered to the wand assembly 102 through the hose 122 (such as an air tube within the hose 122, or through an internal passage of the hose 122 itself).

One or more batteries 180 may also be stowed within the storage chamber 228. For example, three batteries 180 may be within the storage chamber 228.

A power supply 238 is also contained within the storage chamber 228. The power supply 238 may be coupled to the wand assembly 102 through a power cord (such as via a plug and receptacle fitting) to provide power to the wand assembly 102. Further, the power supply 238 may be configured to provide power to the batteries 180 (such as to recharge the batteries 180). The batteries 180 may be secured to the wand assembly 102 and provide power to the wand assembly 102, so that the wand assembly 102 may be used without connection to the power supply 238.

The cooling fan 236 couples to the hose 122 via the port 234. The cooling fan 236 may also include a diverter port that couples to an internal portion of the power supply 238. In this manner, cooling air may be delivered to both the hose 122 (and therefore the wand assembly 102), and the power supply 238, thereby providing cooling to both the wand assembly 102 and the power supply 238.

A hole 240 may be formed through a portion of the case assembly 200. For example, a hole 240 may be formed through a portion of the top wall 210 and sized and shaped to allow the hose 122 to pass therethrough. In this manner, the hose 122 may remain connected to the wand assembly 102 even when the wand assembly 102 is contained within the storage chamber 228 and the cover 202 is closed. Other portions of the hose 122 between the first end 230 and the second end 232 may be secured to the cover 202 by the hose retainer 218, as shown and described with respect to Figure 19.

As shown, the handle 214 may be secured to the top wall 210 of the main body 201. The handle 214 may be configured to retracted into and extend out of the main body 201. For example, the handle 214 may be a telescoping handle.

The wand assembly 102 is removably secured within the storage chamber 228. For example, the wand assembly 102 may be removably secured within the storage chamber 228 by one or more latches, clips, or via an interference fir with a conforming portion of the case assembly 200.

The power supply 238 may be fixed in position within the storage chamber 228. For example, the power supply 238 may be fixed in the storage chamber 228 by one or more fasteners, adhesives, or the like. Optionally, the power supply 238 may be secured in position by one or more latches, clips, or the like.

The batteries 180 may similarly be fixed position within the storage chamber 228. For example, the batteries 180 may be fixed in the storage chamber 228 by one or more fasteners, adhesives, or the like. Optionally, the batteries 180 may be secured in position by one or more latches, clips, or the like. In at least one other example, the batteries 180 may be removable, and configured to couple directly to the wand assembly 102 to provide power thereto.

Figure 22 illustrates a perspective view of the portable sanitizing system 100 having the case assembly 200 in the open position, according to an example of the present disclosure. A power cord 250 may also be stowed within the storage chamber 228. The power cord 250 is contained within the case assembly 200 when the cover 202 is closed and the portable sanitizing system 100 is moved when the wand assembly 102 is not being operated.

Optionally, the power cord 250 connects the power supply 238 to a source of power (such as a wall outlet). In addition to supply air to the wand assembly 102, the hose 122 also routes electrical cables and the like to the wand assembly 102 from the power supply 238 and the batteries 180.

Optionally, the hose 122 may not include electrical connections to the wand assembly 102. Instead, the wand assembly 102, the power cord 250 may plug into the wand assembly 102, via the plug 252, to supply power from the power supply 238 and/or the batteries 180. In this example, as the wand assembly 102 is operated, the plug 252 of the power cord 250 is connected to a reciprocal receptacle of the wand assembly 102. An opposite end of the power cord 250 is connected to the power supply 238 (and/or, a battery 180). The power cord 250 extends out of the case assembly 200 through the hole 240. Thus, the wand assembly 102 may be removed from the storage chamber 228 and connected to the hose 122 and the power cord 250, which extend through the hole 240. The cover 202 may then be closed, thereby securely retaining the power supply 238, the batteries 180, and the like within the storage chamber 228. The wand assembly 102 may then be activated, as it is powered via the power supply 238 or one or more of the batteries 180, and the closed case assembly 200 may be moved, such as via an individual grasping the handle 214 and rolling the case assembly 200 via the casters 216 (shown in Figures 19 and 20).

Further, the hole 240 also allows intake air to be drawn into the storage chamber 228, even when the cover 202 is closed over the main body 201. Accordingly, the cooling fan 236 is able to receive fresh air, even when the cover 202 is closed.

The power supply 238 may be configured to receive power from a standard power supply, such as a source of alternating current power. For example, the power supply 238 may connect to the source of alternating current power through a power cord. The power cord 250 connects to the wand assembly 102, and is configured to deliver power to the wand assembly 102 to operate the UV lamp 140 from power received from the power supply 238 and optionally the batteries 180. For example, when the power supply 238 is connected to a source of alternating current power, the wand assembly 102 is powered by the power supply 238. In the absence of such power, the wand assembly 102 may be powered by the batteries 180. For example, the wand assembly 102 receives power from the batteries 180 the power supply 238 is not plugged into a power outlet. If the power supply 238 is plugged into a power outlet, one or more relays in the power supply 238 switch over from the batteries 180 to alternating current power supply from the power outlet.

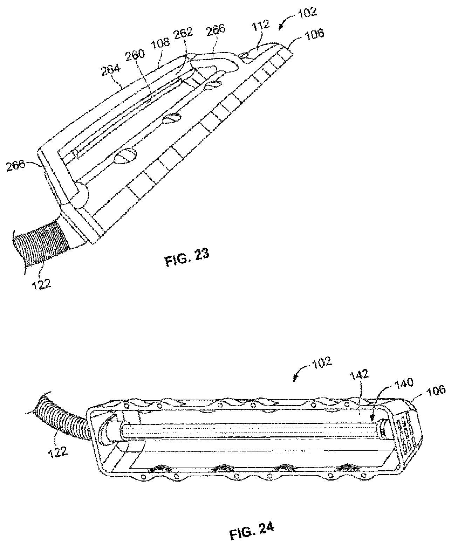

Figure 23 illustrates a perspective lateral view of the wand assembly 102, according to an example of the present disclosure. As shown, the handle 108 may be fixed in relation to the shroud 112. For example, the handle 108 may be integrally molded and formed with the shroud 112. The wand assembly 102 may be small and compact in order to fit in confined spaced, such as within a flight deck of an aircraft.

An activation trigger 260 is moveably coupled to the handle 108. For example, the activation trigger 260 may be secured to an underside 262 of a main beam 264 of the handle 108. The activation trigger 260 is configured to be selectively pressed and/or depressed to activate and deactivate the UV lamp 140 of the wand assembly 102, as desired.

The activation trigger 260 may be located anywhere along the length of the handle 108. The activation trigger 260 may be shaped differently than shown. Further, the activation trigger 260 may be smaller or larger than shown. As an example, the activation trigger 260 may be a circular button, instead of an elongated bar or beam, as shown. Also, optionally, the activation trigger 260 may be located on a top portion of the main beam 264, or on an extension beam 266, which spaces the handle 108 from the shroud 112. As another example, the activation trigger 260 may be located on a portion of the shroud 112.

Figure 24 illustrates a perspective bottom view of the wand assembly 102 of Figure 23. As shown, the reflector 142 is secured to an underside of the shroud 112.

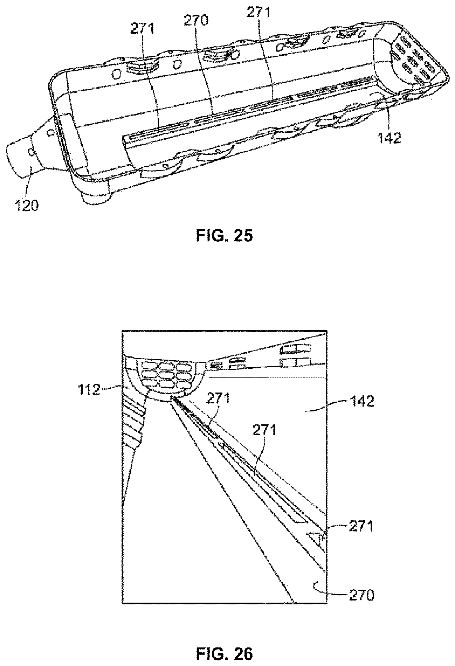

Figure 25 illustrates a perspective bottom view of the wand assembly 102 of Figures 23 and 24 without the UV lamp 140 (for the sake of clarity), according to an example of the present disclosure. Figure 26 illustrates a perspective view of a cooling manifold 270 of the shroud 112 of the wand assembly 102. Referring to Figures 25 and 26, a half of the reflector 142 is removed to expose a cooling manifold 270 that extends through the shroud 112 and is in fluid communication with the port 120. The cooling manifold 270 has a plurality of air outlets 271 that allow air delivered through the hose 122 (shown in Figure 23, in at least one example) that is coupled to the port 120 to pass over the UV lamp 140 when activated. In this manner, the UV lamp 140 is cooled during operation. The delivered air passes over and around the reflector 142 (which is disposed between the cooling manifold 270 and the UV lamp 140), through a channel defined through the reflector 142, and/or between two portions of the reflector 142 (such as a first half of the reflector 142 and a second half of the reflector 142).

Referring to Figures 19-26, the portable sanitizing system 100 includes the wand assembly 102 including the sanitizing head 106 having the UV lamp 140. The case assembly 200 includes the cover 202 coupled to the main body 201. The cover 202 is configured to be moved between an open position that exposes the storage chamber 228 and a closed position. The wand assembly 102 is configured to be stored in the storage chamber 228 when not in use and removed from the storage chamber 228 to disinfect one or more components with UV light emitted by the UV lamp 140.

In at least one example, the portable sanitizing system 100 includes the wand assembly 102 and the case assembly 200, which may be a rolling case assembly. The wand assembly 102 includes the UV lamp 140. The cooling manifold 270 is configured to allow air to blow across the UV lamp 140, such as one or more bulbs of the UV lamp 140. The wand assembly 102 may also include a two piece reflector 142, a master power switch, and a trigger switch, such as the activation trigger 260, to activate and illuminate the UV lamp 140.

During use of the wand assembly 102, the case assembly 200 may be placed away from the area being disinfected, thereby allowing the operator to transport only the wand assembly 102 to the area, and facilitating movement and operation in tight or confined spaces. The wand assembly 102 may include a 300 watt, 222 nm UV lamp, optional ranging lights, the cooling manifold 270 running the length of the shroud 112, the reflector 142, mounts (such as brackets, clamps, fasteners, and/or the like) to secure the UV lamp 140 to the shroud 112, a master power switch on the handle 108, and the activation trigger 260 on the handle 108 that is configured to be engaged to selectively activate and deactivate the UV lamp 140. The reflector 142 may be made out of Teflon or an aluminum sheet, which allows the reflector 142 to provide electromagnetic shielding. The UV lamp 140 may be attached to the shroud 112 with wire straps or bands, which may be positioned on top of Teflon tape and dry woven fiberglass that serve as a cushion between the strap and the glass bulb.

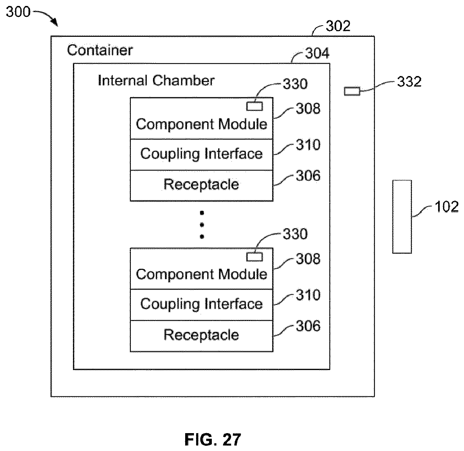

Figure 27 illustrates a schematic block diagram of a portable sanitizing system 300, according to an example of the present disclosure. The portable sanitizing system 300 includes a container 302 that defines or otherwise includes an internal chamber 304. The backpack assembly 104 shown in Figures 1, 5-8, and 16-17 is an example of the container 302. The case assembly 200 shown in Figures 19-22 is another example of the container 302. A cart assembly, such as shown and described in United States Patent Application 17/026,435, entitled "Ultraviolet Light Sanitizing Cart," filed September 21, 2020, is another example of the container 302.

The internal chamber 304 includes a plurality of receptacles 306 or other such mounting locations that are configured to receive and removably retain a plurality of component modules 308. Examples of the component modules 308 includes a power supply, a battery pack, a blower or fan, a wand assembly, and/or the like, as described herein. The internal chamber 304 can include any number of receptacle 306, each of which is configured to removably retain a respective component module 308.

A wand assembly 102 is coupled to the container 302. The wand assembly 102 can be any of the wand assemblies described herein. The wand assembly 102 can be stowed within the internal chamber 304, and removed therefrom to sanitize surfaces with UV light, as described herein. In at least one example, the wand assembly 102 is a component module 308 that is removably coupled to a receptacle 306 within the internal chamber 304.

The component modules 308 secure to the receptacles 306 at respective coupling interfaces 310. The component modules 308 are configured to be secured to, and removed from, the receptacles 306 at the coupling interfaces 310, such as without the use of tools. The component modules 308 are removably secured to the container 302, such as with respect the receptacles 306. The component modules 308 are removably secured to the container 302 in that they are configured to be selectively and repeatedly secured to and removed from the container 302, such as without the use of tools. As an example, the coupling interfaces 310 include fasteners that are configured to be manipulated by hand, such as quarter turn wing-style fasteners. As another example, the coupling interfaces 310 include plug and socket connections. As another example, the coupling interfaces 310 include latches. As another example, the coupling interfaces 310 includes deflectable detents. As another example, the coupling interface 310 include snaps. As another example, the coupling interfaces 310 include mechanical and electrical couplings, such as plugs and sockets.

The component modules 308 are configured to be removably secured within the container 302, such as via the coupling interfaces 310. As such, the component modules 308 can be quickly and easily removed, repaired outside of the container 302, and/or replaced with another component module. Further, the component modules 308 can be removed from a first container, such as of a case assembly, a backpack assembly, or a cart assembly, and inserted into a second container that differs from the first container, such as another one of a case assembly, a backpack assembly, or a cart assembly

The component modules 308 can be interchangeable with respect to the receptacles 306 within the container 302. The coupling interfaces 310 can be the same for each of the component modules 308 and the receptacles 306. For example, the coupling interfaces 310 can be standardized to provide a common securing connection for all of the component modules 308. As such, a first component module 308 within the container 302 can be removed from a first receptacle 306, and moved to a second receptacle 306 within the container 302. Additionally, component modules 308 can be interchanged within the container 302 and/or another container with different component modules 308.

In at least one other example, the sanitizing system may not be portable. Instead, the sanitizing system can be fixed in a location, such as within a galley, lavatory, or the like of a vehicle. The fixed sanitizing system can include the container 302.

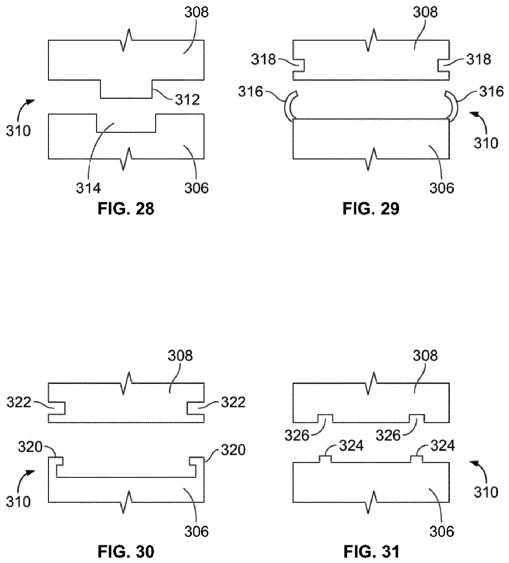

Figure 28 illustrates a simplified view of a coupling interface 310 between a component module 308 and a receptacle 306, according to an example of the present disclosure. As shown, the coupling interface 310 includes a plug 312 that mates with a reciprocal socket 314 of the receptacle 306. When mated together, the plug 312 and the socket 314 provide a mechanical connection that secures the component module 308 to the receptacle 306. Further, the plug 312 and the socket 314 may also include electrical elements that provide an electrical connection between the component module 308 and the receptacle 306 when the plug 312 is mated with the socket 314. Optionally, the receptacle 306 can include the plug 312, and the component module 308 can include the receptacle 314.

Figure 29 illustrates a simplified view of a coupling interface 310 between a component module 308 and a receptacle 306, according to an example of the present disclosure. As shown, the coupling interface 310 includes one or more latches 316 of the receptacle 306 that are configured to latchably engage one or more strikes 318, of the component module 308. Optionally, the receptacle 306 can include the strike(s) 318, and the component module 308 can include the latch(es) 316.

Figure 30 illustrates a simplified view of a coupling interface 310 between a component module 308 and a receptacle 306, according to an example of the present disclosure. As shown, the coupling interface 310 includes one or more detents 320 (such as deflectable arms, spring-biased devices, or the like) of the receptacle 306 that are configured to deflect, and mate with one or more reciprocal members 322 (such as recesses, ledges, ridges, or the like) of the component module 308. Optionally, the receptacle 306 can include the reciprocal member(s) 322 and the component module 308 can include the detent(s) 320.

Figure 31 illustrates a simplified view of a coupling interface between a component module and a receptacle, according to an example of the present disclosure. As shown, the coupling interface 310 includes one or more snaps 324 of the receptacle 306 that are configured to snapably secured into one or more reciprocal recesses 326 of the component module 308. Optionally, the receptacle 306 can include the recess(es) 326 and the component module 308 can include the snap(s) 324.

Figures 28-31 show examples of coupling interfaces 310. Various other types of coupling interfaces 310 that allow the component modules 308 to be removably secured to the receptacles 306 can be used.

Referring to Figures 27-31, the component modules 308 of the portable sanitizing system 300 are configured for quick and easy removal and replacement. Spare component modules 308 can be stored to ensure that replacements can be quickly accomplished in the field, to maintain operational schedules. The component modules 308, including replacement component modules, can include radio frequency identification (RFID) tags 330. The RFID rags 330 allow the component modules 308 to be tracked and monitored. As such, inventory can be efficiently managed, the number of component modules 308 can be optimized for efficiency and cost benefits. Optionally, the component modules 308 may not include RFID tags.

The component modules 308 are secured to the container 302 via the coupling interfaces 310, such as may include quick-release fasteners and quick-release electrical connectors, which require little or no tools to secure and remove the component modules 308. In at least one example, the portable sanitizing system 300 can be tracked and monitored via a global positioning system (GPS) device 332, using a GPS-enabled asset management system. The asset management system can provide asset status (battery state, remote diagnostics, etc.) and geographic location wirelessly to a control center and/or a remote computing device (such as handheld smart phone, smart tablet, personal or laptop computer, or the like). As such, efficiency can be improved by ensuring the portable sanitizing system 300 is fully functional when and where needed. Optionally, the container 302 may not include the GPS device.

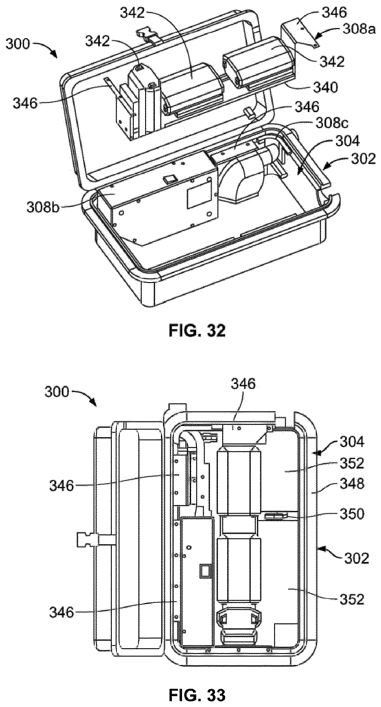

Figure 32 illustrates a perspective view of the portable sanitizing system 300, according to an example of the present disclosure. Figure 33 illustrates a top view of the portable sanitizing system 300 of Figure 32. Referring to Figures 32 and 33, as shown, the container 302 is a case assembly. The component modules include a battery pack 308a, a power supply 308b, and a blower 308c. The battery pack 308a includes a support tray 340 that securely retains one or more batteries 342. The battery pack 308a can include more or less batteries 342 than shown.

As an example, the batteries 342 provide direct current (DC) power, such as 120 V DC power. The power supply 308b uses 110 alternating current (AC) power, such as may be provided from a main power source within a vehicle, building, or the like. For example, the power supply 308b can connect to the main power source through an electrical cord and plug. In at least one example, the power supply 308b converts power from the main power source and/or the batteries 342 to a high voltage, high frequency power, such as 3000-5000 V at a frequency of 120 kHz.

As shown, the battery pack 308a, the power supply 308b, and the blower 308c include coupling interfaces that include flanges 346 that seat over portions of a ledge 348 of the container 302. The flanges 346 secure to the ledge 348 such as through any of the coupling interfaces described with respect to Figures 27-31. For example, quarter turn, hand engageable fasteners can be used to secure the flanges 346 to the ledge 348.

Additionally, a coupling interface 350 can be used to quickly connect and disconnect the batteries 342 in relation to the power supply 308b. The coupling interface 350 can be a plug/socket electrical connector, in at least one example.

Additionally, the container 302 can include a bed 352 configured to support a wand assembly. The bed 352 can be formed of foam, in at least one example.

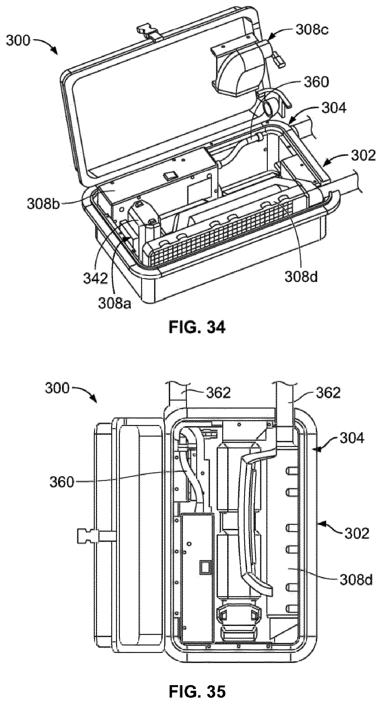

Figure 34 illustrates a perspective view of the portable sanitizing system 300, according to an example of the present disclosure. Figure 35 illustrates a top view of the portable sanitizing system 300 of Figure 34. An electrical connector 360 extends from the power supply 308b, and is configured to removably connect to a wand assembly 308d through a reciprocal mating interface extending through a hose 362 that connects to the wand assembly 308d. The electrical connector 360 can also removably connect to the blower 308c through a reciprocal mating interface on and/or within the blower 308c.

Referring to Figures 32-35, the various component modules, such as the battery pack 308a, the power supply 308b, the blower 308c, and/or the wand assembly 308d can be secured within the container 302 and removed therefrom, as described with respect to Figures 27-35.

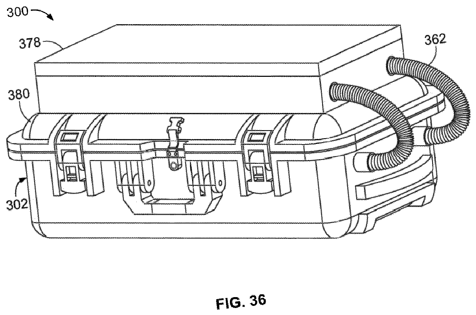

Figure 36 illustrates a perspective side of a portable sanitizing system 300, according to an example of the present disclosure. In at least one example, a retaining frame 378 can extend outwardly from a cover 380 of the container 302. The retaining frame 378 is configured to house supplemental or replacement component modules, such as additional battery packs. Additionally, portions of the hose 362 can be secured within or underneath the retaining frame 378 such as when the portable sanitizing system 300 is stowed away.

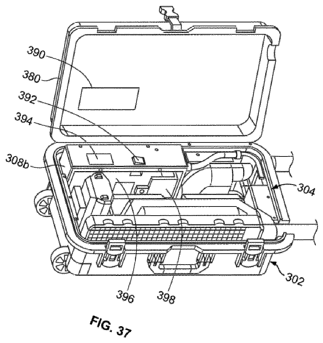

Figure 37 illustrates a perspective top view of a portable sanitizing system 300, according to an example of the present disclosure. In at least one example, a window 390 is disposed within the cover 380. The window 390 can be an opening formed through the cover 380. As another example, the window 390 can be a transparent material disposed within, or formed as a portion of, the cover 380. For example, the window 390 can be formed of glass, a clear polycarbonate material, or the like. The window 390 is configured to provide a clear view into a portion of the internal chamber 304 when the cover 380 is closed, in at least one example.

For example, the power supply 308b can include an activation switch 392 that illuminates when the power supply 308b is activated. An information screen 394 can also be disposed on the power supply 308b. The information screen 394 can be an electric monitor, such as including a liquid crystal display, light emitting diodes (LEDs), and/or the like that provide various status indicators for the portable sanitizing system 300. The status indicators can include an activation status, a power level, diagnostic information, such as battery life remaining, UV lamp temperature and status, and/or the like.

The window 390 is positioned to be over the activation switch 392 and the information screen 394 when the cover 380 is closed. As such, the window 390 allows an individual to view the activation switch 392 and the information screen 394 when the cover 380 is closed.

In at least one example, a printed circuit board 396 can also be disposed within the internal chamber 304. For example, the printed circuit board 396 can be secured on and/or proximate to the power supply 308b. The printed circuit board 396 includes a microcontroller 398. In at least one example, the printed circuit board 396 is, or is part of, a component module.

In at least one example, the microcontroller 398 is configured to detect information regarding the component modules. For example, the microcontroller 398 detects a make and model of attached component modules, such as the wand assembly 308d, the battery pack 308a, the power supply 308b, the blower 308d, and/or the like. The microcontroller 398 is in communication with the various component modules, such as through one or more wired or wireless connections, when the component modules are plugged into the container 302, in at least one example.

In at least one example, the microcontroller 398 detects information regarding the a component module (such as make and model), based on a connector pin arrangement of the component module. The microcontroller 398 compares installed pin arraignment data to known (such as stored in memory) arrangements to determine compatibility of the component module. The microcontroller 398 outputs data signals that include messages, which may be displayed on the information screen 394. The messages are displayed on the information screen 394 in response to the pin arrangement comparison, in at least one example. In at least one example, the microcontroller 398 can further adjust system parameters, such as power level, to optimize the portable sanitizing system 300 with respect to particular component modules secured to the container 302. Optionally, the portable sanitizing system 300 may not include the window 390, the information screen 394, the printed circuit board 396, or the microcontroller 398.

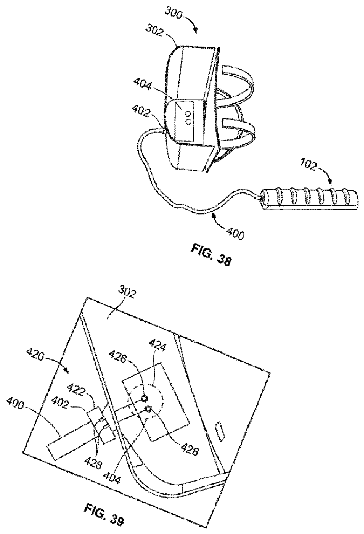

Figure 38 illustrates a perspective lateral view of a portable sanitizing system 300, according to an example of the present disclosure. As shown, the container 302 can be a backpack assembly. Optionally, the container 302 can be a case assembly, a cart assembly, or the like.

The wand assembly 102 can be a component module. The wand assembly 102 connects to a hose 400, which includes electrical wiring. The hose 400 can also include a fluid duct, such as an air tube. A first connector 402 is disposed at an end of the hose 400 opposite from the wand assembly 102. The first connector 402 is configured to removably connect to a second connector 404 on the container 302. As such, the first connector 402 and the second connector 404 provide a coupling interface.

The first connector 402 mates with the second connector 404 to provide a mechanical and electrical connection between the hose 400 (and therefore the wand assembly 102) and the container 302. The wand assembly 102 can be quickly and easily connected to and disconnected from the container 302 via the coupling interface between the first connector 402 and the second connector 404.

Figure 39 illustrates a simplified view of a coupling interface 420 between the hose 400 and the container 302, according to an example of the present disclosure. The first connector 402 includes a threaded retainer 422 that threadably secures to a reciprocal member 424 of the second connector 404. The second connector 404 also includes one or more conductive receivers 426 that receive one or more conductive rods 428 of the first connector 402, such as through a slip fit. The threaded retainer 422 is threaded around the reciprocal member 424 to securely and safely mate the rods 428 with the receivers 426. Thus, the threaded hose retainer 422 can be rotated to selectively connect and disconnect the first connector 402 from the second connector 404.

In order to secure the hose 400 to the container 302, the threaded retainer 422 is aligned with the reciprocal member 424 so that the rods 428 are aligned with the receivers 426. The hose 400 is then urged toward the reciprocal member 424 so that the rods 428 are inserted into the receivers 426. The threaded retainer 422 is then rotated in relation to the reciprocal member in a securing direction to threadably secure the first connector 402 to the second connector 404. In order to remove the hose 400 from the container 302, the process is reversed.

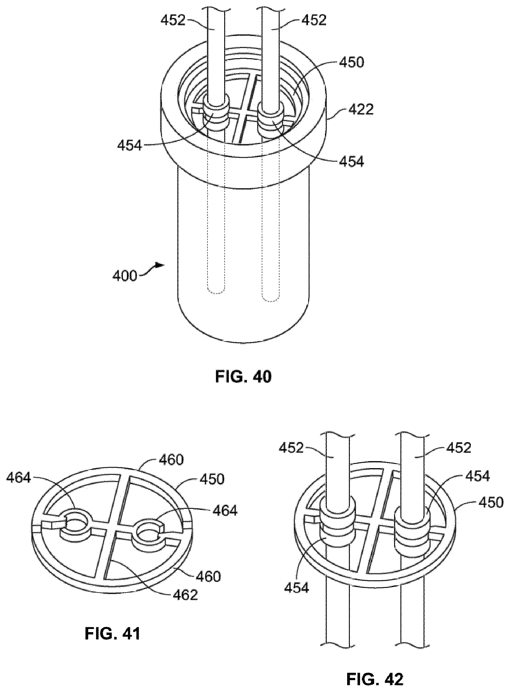

Figure 40 illustrates a perspective end view of a hose, according to an example of the present disclosure. A wire retainer 450 is disposed within the hose 400 and is configured to retain electrical wires 452. Strain reliefs 454 may be disposed between the wire retainer 450 and the electrical wires 452.

Figure 41 illustrates a perspective top view of the wire retainer 450, according to an example of the present disclosure. Figure 42 illustrates a perspective view of the electrical wires 452 retained by the wire retainer 450. Referring to Figures 40-42, the wire retainer 450 includes an outer annular rim 460 that is sized and shaped to conform to an interior surface of the hose 400. The wire retainer 450 is inside the hose 400. A cross beam 462 spans across segments of the rim 460. Hose couplers 464 extend from the cross beam 462 inside of the annular rim 460. The hose couplers 464 are sized and shaped to secure around outer surfaces of the electrical wires 452.

The strain reliefs 454 can be above and below the hose couplers 464. The strain reliefs 454 can be bonded to the electrical wires 452 after the electrical wires 452 are inserted into the hose couplers 464. Optionally, the electrical wires 452 may be retained within the hose 400 through different wire retainers 450 or even without wire retainers 450.

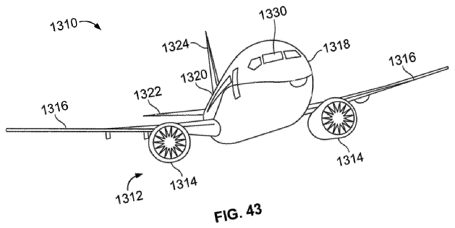

Figure 43 illustrates a perspective front view of an aircraft 1310, according to an example of the present disclosure. The aircraft 1310 includes a propulsion system 1312 that includes engines 1314, in at least one example. Optionally, the propulsion system 1312 may include more engines 1314 than shown. The engines 1314 are carried by wings 1316 of the aircraft 1310. In other examples, the engines 1314 may be carried by a fuselage 1318 and/or an empennage 1320. The empennage 1320 may also support horizontal stabilizers 1322 and a vertical stabilizer 1324.

The fuselage 1318 of the aircraft 1310 defines an internal cabin 1330, which includes a flight deck or cockpit, one or more work sections (in at least one example, galleys, personnel carry-on baggage areas, and the like), one or more passenger sections (in at least one example, first class, business class, and coach sections), one or more lavatories, and/or the like. The internal cabin 1330 includes one or more lavatory systems, lavatory units, or lavatories, as described herein.

Alternatively, instead of an aircraft, examples of the present disclosure may be used with various other vehicles, such as automobiles, buses, locomotives and train cars, watercraft, and the like. Further, examples of the present disclosure may be used with respect to fixed structures, such as commercial and residential buildings.

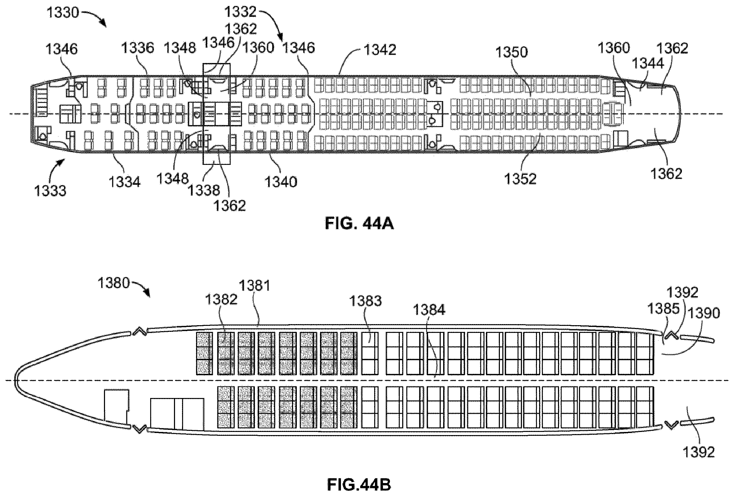

Figure 44A illustrates a top plan view of an internal cabin 1330 of an aircraft, according to an example of the present disclosure. The internal cabin 1330 may be within the fuselage 1332 of the aircraft, such as the fuselage 318 of Figure 43. For example, one or more fuselage walls may define the internal cabin 1330. The internal cabin 1330 includes multiple sections, including a front section 1333, a first class section 1334, a business class section 1336, a front galley station 1338, an expanded economy or coach section 1340, a standard economy of coach section 1342, and an aft section 1344, which may include multiple lavatories and galley stations. It is to be understood that the internal cabin 1330 may include more or less sections than shown. For example, the internal cabin 1330 may not include a first class section, and may include more or less galley stations than shown. Each of the sections may be separated by a cabin transition area 1346, which may include class divider assemblies between aisles 1348.

As shown in Figure 44A, the internal cabin 1330 includes two aisles 1350 and 1352 that lead to the aft section 344. Optionally, the internal cabin 1330 may have less or more aisles than shown. For example, the internal cabin 1330 may include a single aisle that extends through the center of the internal cabin 1330 that leads to the aft section 1344.

The aisles 1348, 1350, and 1352 extend to egress paths or door passageways 1360. Exit doors 1362 are located at ends of the egress paths 1360. The egress paths 1360 may be perpendicular to the aisles 1348, 1350, and 1352. The internal cabin 1330 may include more egress paths 1360 at different locations than shown. The portable sanitizing systems shown and described with respect to Figures 1-42 may be used to sanitize various structures within the internal cabin 1330, such as passenger seats, monuments, stowage bin assemblies, components on and within lavatories, galley equipment and components, and/or the like.

Figure 44B illustrates a top plan view of an internal cabin 1380 of an aircraft, according to an example of the present disclosure. The internal cabin 1380 is an example of the internal cabin 1330 shown in Figure 43. The internal cabin 1380 may be within a fuselage 1381 of the aircraft. For example, one or more fuselage walls may define the internal cabin 1380. The internal cabin 1380 includes multiple sections, including a main cabin 1382 having passenger seats 1383, and an aft section 1385 behind the main cabin 1382. It is to be understood that the internal cabin 1380 may include more or less sections than shown.

The internal cabin 1380 may include a single aisle 1384 that leads to the aft section 1385. The single aisle 1384 may extend through the center of the internal cabin 1380 that leads to the aft section 1385. For example, the single aisle 1384 may be coaxially aligned with a central longitudinal plane of the internal cabin 1380.

The aisle 1384 extends to an egress path or door passageway 1390. Exit doors 1392 are located at ends of the egress path 1390. The egress path 1390 may be perpendicular to the aisle 1384. The internal cabin 1380 may include more egress paths than shown. The portable sanitizing systems shown and described with respect to Figures 1-42 may be used to sanitize various structures within the internal cabin 1330, such as passenger seats, monuments, stowage bin assemblies, components on and within lavatories, galley equipment and components, and/or the like.

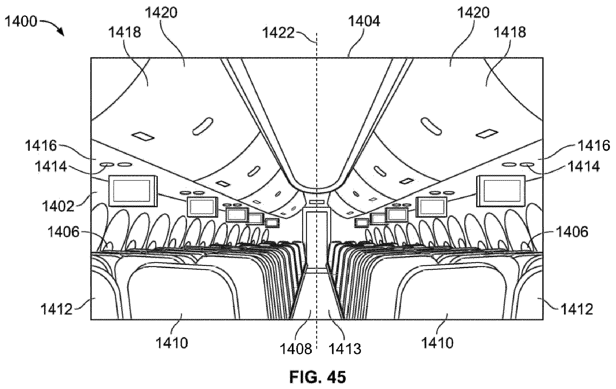

Figure 45 illustrates a perspective interior view of an internal cabin 1400 of an aircraft, according to an example of the present disclosure. The internal cabin 1400 includes outboard walls 1402 connected to a ceiling 1404. Windows 1406 may be formed within the outboard walls 1402. A floor 1408 supports rows of seats 1410. As shown in Figure 45, a row 1412 may include two seats 1410 on either side of an aisle 1413. However, the row 1412 may include more or less seats 1410 than shown. Additionally, the internal cabin 1400 may include more aisles than shown.

Passenger service units (PSUs) 1414 are secured between an outboard wall 1402 and the ceiling 1404 on either side of the aisle 1413. The PSUs 1414 extend between a front end and rear end of the internal cabin 1400. For example, a PSU 1414 may be positioned over each seat 1410 within a row 1412. Each PSU 1414 may include a housing 1416 that generally contains vents, reading lights, an oxygen bag drop panel, an attendant request button, and other such controls over each seat 1410 (or groups of seats) within a row 1412.

Overhead stowage bin assemblies 1418 are secured to the ceiling 1404 and/or the outboard wall 1402 above and inboard from the PSU 1414 on either side of the aisle 1413. The overhead stowage bin assemblies 1418 are secured over the seats 1410. The overhead stowage bin assemblies 1418 extend between the front and rear end of the internal cabin 1400. Each stowage bin assembly 1418 may include a pivot bin or bucket 1420 pivotally secured to a strongback (hidden from view in Figure 45). The overhead stowage bin assemblies 1418 may be positioned above and inboard from lower surfaces of the PSUs 1414. The overhead stowage bin assemblies 1418 are configured to be pivoted open in order to receive passenger carry-on baggage and personal items, in at least one example.

As used herein, the term "outboard" means a position that is further away from a central longitudinal plane 1422 of the internal cabin 1400 as compared to another component. The term "inboard" means a position that is closer to the central longitudinal plane 1422 of the internal cabin 1400 as compared to another component. For example, a lower surface of a PSU 1414 may be outboard in relation to a stowage bin assembly 1418.

The portable sanitizing systems shown and described with respect to Figures 1-42 may be used to sanitize various structures shown within the internal cabin 1400.

When not in use, the portable sanitizing systems may be stored within a closet, galley cart bay, or galley cart, such as within the internal cabin of the vehicle.

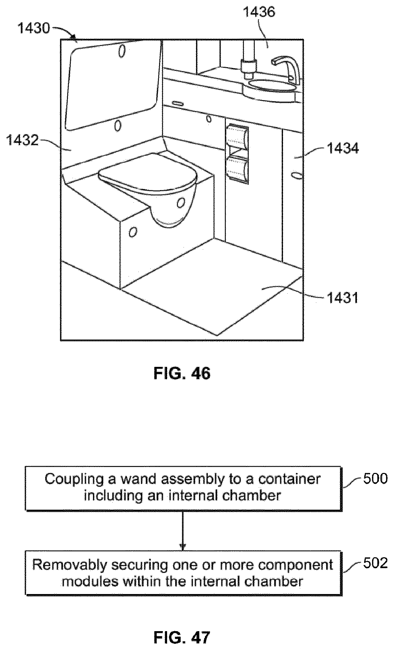

Figure 46 illustrates a perspective internal view of a lavatory 1430 within an internal cabin of a vehicle, such as any of the internal cabins described herein. The lavatory 1430 is an example of an enclosed space, monument or chamber, such as within the internal cabin a vehicle. The lavatory 1430 may be onboard an aircraft, as described above. Optionally, the lavatory 1430 may be onboard various other vehicles. In other examples, the lavatory 1430 may be within a fixed structure, such as a commercial or residential building. The lavatory 1430 includes a base floor 1431 that supports a toilet 1432, cabinets 1434, and a sink 1436 or wash basin. The lavatory 1430 may be arranged differently than shown. The lavatory 1430 may include more or less components than shown. The portable sanitizing systems shown and described with respect to Figures 1-42 may be used to sanitize the various structures, components, and surfaces within the lavatory 1430.

The portable sanitizing systems as described herein can be used to safely and effectively sanitize high-touch surfaces in the flight deck and internal cabin in a timely and cost-effective manner. UV disinfection allows the internal cabin to be quickly and effectively disinfected, such as between flights. In at least one example, the portable sanitizing systems are used to augment a cleaning process, such as after manual cleaning.

Figure 47 illustrates a flow chart of a portable sanitizing method, according to a non-claimed example. The method include coupling, at 500, a wand assembly to a container including an internal chamber. The wand assembly includes a sanitizing head having an ultraviolet (UV) lamp. The method further includes removably securing, at 502, one or more component modules within the internal chamber.

In at least one example, said removably securing includes removably securing the one or more component modules to one or more receptacles of the container through one or more coupling interfaces.

In at least one example, the method further includes disposing a radio frequency identification (RFID) tag on or within the container.

In at least one example, the method also includes disposing a global positioning system (GPS) device on or within the container.

In at least one example, the method also includes disposing a window on a portion of the container.

In at least one example, the one or more component modules includes a power supply. The portable sanitizing method further includes displaying status indicators on an information screen of the power supply.

As an example, the method also includes detecting, by a microcontroller of the container, information regarding the one or more component modules.

In at least one example, the method also includes connecting the wand assembly to the container with a hose, and removably connecting a first connector of the hose to a second connector of the container.

In at least one example, the method may be used with fixed sanitizing systems. For example, the method may be used with respect to fixed UV sanitizing systems that include UV lamps, whether or not within a wand assembly.

As described herein, examples of the present disclosure provide systems and a methods for efficiently sterilizing surfaces, components, structures, and/or the like within an internal cabin of a vehicle. Further, examples of the present disclosure provide compact, easy-to-use, and safe systems and methods for using UV light to sterilize surfaces within an internal cabin.

As described herein, examples of the present disclosure provide systems and methods for quickly and easily repairing or replacing components of UV sanitizing systems. Further, examples of the present disclosure provide UV sanitizing system that can be easily adapted for different needs.

While various spatial and directional terms, such as top, bottom, lower, mid, lateral, horizontal, vertical, front and the like can be used to describe examples of the present disclosure, it is understood that such terms are merely used with respect to the orientations shown in the drawings. The orientations can be inverted, rotated, or otherwise changed, such that an upper portion is a lower portion, and vice versa, horizontal becomes vertical, and the like.

As used herein, a structure, limitation, or element that is "configured to" perform a task or operation is particularly structurally formed, constructed, or adapted in a manner corresponding to the task or operation. For purposes of clarity and the avoidance of doubt, an object that is merely capable of being modified to perform the task or operation is not "configured to" perform the task or operation as used herein.

It is to be understood that the above description is intended to be illustrative, and not restrictive. For example, the above-described examples (and/or aspects thereof) can be used in combination with each other. In addition, many modifications can be made to adapt a particular situation or material to the teachings of the various examples of the disclosure without departing from the scope of the claims. While the dimensions and types of materials described herein are intended to define the parameters of the various examples of the disclosure, the examples are by no means limiting and are exemplary examples. Many other examples will be apparent to those of skill in the art upon reviewing the above description. The scope of the various examples of the disclosure should, therefore, be determined with reference to the appended claims, along with the full scope of equivalents to which such claims are entitled. In the appended claims and the detailed description herein, the terms "including" and "in which" are used as the plain-English equivalents of the respective terms "comprising" and "wherein." Moreover, the terms "first," "second," and "third," etc. are used merely as labels, and are not intended to impose numerical requirements on their objects.

## Claims

1. A portable sanitizing system (100), comprising:
a wand assembly (102) comprising a sanitizing head (106) having an ultraviolet (UV) lamp (140);
a container comprising an internal chamber (178), wherein the wand assembly (102) is coupled to the container; and
one or more component modules removably secured within the internal chamber (178),
wherein the container further comprises a microcontroller (398) configured to detect, based on a connector pin arrangement, make and model of the one or more component modules, wherein the microcontroller (398) compares the installed connector pin arrangement data of one or more component modules to known arrangements stored in a memory to determine compatibility of the one or more component modules.

2. The portable sanitizing system (100) of claim 1, wherein the container is a backpack assembly (104) or a case assembly.

3. The portable sanitizing system (100) of claim 1, wherein the one or more component modules comprise a power supply (380b), a battery pack (308a), or a blower (308d).

4. The portable sanitizing system (100) of any of claims 1-3, wherein the one or more component modules include the wand assembly (102).

5. The portable sanitizing system (100) of claim 1-3, wherein the container further comprises one or more receptacles within the internal chamber (178), and wherein the one or more component modules are configured to removably secure to the one or more receptacles through one or more coupling interfaces.

6. The portable sanitizing system (100) of claim 5, wherein the one or more coupling interfaces comprise a plug and a socket, one or more latches, one or more detents, or one or more snaps.

7. The portable sanitizing system (100) of any of claims 1-3, wherein the container further comprises a radio frequency identification (RFID) tag.

8. The portable sanitizing system (100) of any of claims 1-3, further comprising a retaining frame secured to the container, wherein the retaining frame is configured to house one or more additional component modules.

9. The portable sanitizing system (100) of any of claims 1-3, wherein the container further comprises a window (390) that is configured to allow viewing into at least a portion of the internal chamber (178).

10. The portable sanitizing system (100) of any of claims 1-3, wherein the one or more component modules comprises a power supply (308b), wherein the power supply (308b) comprises an activation switch (392) and an information screen (394), and wherein status indicators are displayed on the information screen (394).

11. The portable sanitizing system (100) of any of claims 1-10, wherein the container further comprises a global positioning system (GPS) device.

12. The portable sanitizing system (100) of claim 3, wherein the microcontroller (398) is configured to detect a make and model of the one or more secured power supply (308b), battery pack (308a), and/or the blower (308d).

13. The portable sanitizing system (100) of any of claims 1-3, further comprising a hose (122) that connects the wand assembly (102) to the container, wherein the hose (122) comprises a first connector that removably connects to a second connector of the container.

14. A portable sanitizing system (100), comprising:
a container comprising:
an internal chamber (178);
a window (390) that is configured to allow viewing into at least a portion of the internal chamber (178);
a microcontroller (398);
a first connector; and
component modules removably secured within the internal chamber (178), wherein the microcontroller (398) is configured to detect, based on a connector pin arrangement, make and model of the one or more component modules, wherein the microcontroller (398) compares the installed connector pin arrangement data of one or more component modules to known arrangements stored in a memory to determine compatibility of the one or more component modules and wherein the component modules comprise:
a wand assembly (102) comprising a sanitizing head (106) having an ultraviolet (UV) lamp (140);
a power supply (380b) including an activation switch and an information screen (394), wherein status indicators are displayed on the information screen (394);
a battery pack (308a); and
a blower (308d); and
a hose (122) that connects the wand assembly (102) to the container, wherein the hose (122) comprises a second connector that removably connects to the first connector of the container.

## Patentansprüche

1. Tragbares Desinfektionssystem (100), das aufweist:
eine Stabanordnung (102), die einen Desinfektionskopf (106) mit einer Ultraviolett-(UV)-Lampe (140) aufweist;
einen Behälter, der eine Innenkammer (178) aufweist, wobei die Stabanordnung (102) mit dem Behälter gekoppelt ist; und
ein oder mehrere Komponentenmodule, die abnehmbar in der Innenkammer (178) befestigt sind,
wobei der Behälter ferner einen Mikrocontroller (398) aufweist, der konfiguriert ist, basierend auf einer Anschlussstiftanordnung Machart und Modell des einen oder der mehreren Komponentenmodule zu erkennen, wobei der Mikrocontroller (398) die installierten Anschlussstiftanordnungsdaten des einen oder der mehreren Komponentenmodule mit bekannten Anordnungen vergleicht, die in einem Speicher gespeichert sind, um eine Kompatibilität des einen oder der mehreren Komponentenmodule zu bestimmen.

2. Tragbares Desinfektionssystem (100) nach Anspruch 1, wobei der Behälter eine Rucksackanordnung (104) oder eine Kofferanordnung ist.

3. Tragbares Desinfektionssystem (100) nach Anspruch 1, wobei das eine oder die mehreren Komponentenmodule eine Stromversorgung (380b), ein Batteriepack (308a) oder ein Gebläse (308d) aufweist.

4. Tragbares Desinfektionssystem (100) nach einem der Ansprüche 1 bis 3, wobei das eine oder die mehreren Komponentenmodule die Stabanordnung (102) enthalten.

5. Tragbares Desinfektionssystem (100) nach Anspruch 1 bis 3, wobei der Behälter ferner eine oder mehrere Aufnahmen in der Innenkammer (178) aufweist und wobei das eine oder die mehreren Komponentenmodule konfiguriert sind, über eine oder mehrere Kopplungsschnittstellen abnehmbar an der einen oder den mehreren Aufnahmen befestigt zu werden.

6. Tragbares Desinfektionssystem (100) nach Anspruch 5, wobei die eine oder die mehreren Kopplungsschnittstellen einen Stecker und eine Buchse, eine oder mehrere Verriegelungen, eine oder mehrere Rasten oder einen oder mehrere Druckknöpfe aufweisen.

7. Tragbares Desinfektionssystem (100) nach einem der Ansprüche 1 bis 3, wobei der Behälter ferner ein Funkfrequenz-Identifikations- (RFID) Etikett aufweist.

8. Tragbares Desinfektionssystem (100) nach einem der Ansprüche 1 bis 3, das ferner einen an dem Behälter befestigten Halterahmen aufweist, wobei der Halterahmen konfiguriert ist, ein oder mehrere zusätzliche Komponentenmodule aufzunehmen.

9. Tragbares Desinfektionssystem (100) nach einem der Ansprüche 1 bis 3, wobei der Behälter ferner ein Fenster (390) aufweist, das konfiguriert ist, einen Blick in mindestens einen Abschnitt der Innenkammer (178) zu ermöglichen.

10. Tragbares Desinfektionssystem (100) nach einem der Ansprüche 1 bis 3, wobei das eine oder die mehreren Komponentenmodule eine Stromversorgung (308b) aufweisen,
wobei die Stromversorgung (308b) einen Aktivierungsschalter (392) und einen Informationsbildschirm (394) aufweist, und wobei Statusanzeigen auf dem Informationsbildschirm (394) angezeigt werden.

11. Tragbares Desinfektionssystem (100) nach einem der Ansprüche 1 bis 10, wobei der Behälter ferner eine globale Positionsbestimmungssystem (GPS) Vorrichtung aufweist.

12. Tragbares Desinfektionssystem (100) nach Anspruch 3, wobei der Mikrocontroller (398) konfiguriert ist, eine Machart und ein Modell des oder der befestigten Stromversorgung (308b), des Batteriepacks (308a) und/oder des Gebläses (308d) zu erkennen.

13. Tragbares Desinfektionssystem (100) nach einem der Ansprüche 1 bis 3, das ferner einen Schlauch (122) aufweist, der die Stabanordnung (102) mit dem Behälter verbindet, wobei der Schlauch (122) einen ersten Verbinder aufweist, der abnehmbar mit einem zweiten Verbinder des Behälters verbunden ist.

14. Tragbares Desinfektionssystem (100), das aufweist:
einen Behälter, der aufweist:
eine Innenkammer (178);
ein Fenster (390), das konfiguriert ist, einen Blick in mindestens einen Abschnitt der Innenkammer (178) zu ermöglichen;
einen Mikrocontroller (398);
einen ersten Verbinder; und
Komponentenmodule, die abnehmbar in der Innenkammer (178) befestigt sind,
wobei der Mikrocontroller (398) konfiguriert ist, basierend auf einer Anschlussstiftanordnung, Machart und Modell des einen oder der mehreren Komponentenmodule zu erkennen, wobei der Mikrocontroller (398) die installierten Anschlussstiftanordnungsdaten eines oder mehrerer Komponentenmodule mit bekannten Anordnungen vergleicht, die in einem Speicher gespeichert sind, um eine Kompatibilität des einen oder der mehreren Komponentenmodule zu bestimmen, und wobei die Komponentenmodule aufweisen:
eine Stabanordnung (102), die einen Desinfektionskopf (106) mit einer Ultraviolett-(UV)-Lampe (140) aufweist;
eine Stromversorgung (380b), die einen Aktivierungsschalter und einen Informationsbildschirm (394) aufweist, wobei Statusanzeigen auf dem Informationsbildschirm (394) angezeigt werden;
einen Batteriepack (308a); und
ein Gebläse (308d); und
einen Schlauch (122), der die Stabanordnung (102) mit dem Behälter verbindet,
wobei der Schlauch (122) einen zweiten Verbinder aufweist, der abnehmbar mit dem ersten Verbinder des Behälters verbunden ist.

## Revendications

1. Système d'assainissement portable (100), comprenant :
un ensemble perche (102) comprenant une tête d'assainissement (106) présentant une lampe à ultraviolets (UV) (140) ;
un contenant comprenant une chambre interne (178), dans lequel l'ensemble perche (102) est couplé au contenant ; et
un ou plusieurs modules composants fixés de manière amovible à l'intérieur de la chambre interne (178),
dans lequel le contenant comprend en outre un microcontrôleur (398) configuré pour détecter, sur la base d'un agencement de broches de connexion, la marque et le modèle des un ou plusieurs modules composants, dans lequel le microcontrôleur (398) compare les données d'agencement de broches de connexion installées d'un ou plusieurs modules composants à des agencements connus stockés dans une mémoire pour déterminer la compatibilité des un ou plusieurs modules composants.

2. Système d'assainissement portable (100) selon la revendication 1, dans lequel le contenant est un ensemble sac à dos (104) ou un ensemble boîtier.

3. Système d'assainissement portable (100) selon la revendication 1, dans lequel les un ou plusieurs modules composants comprennent une alimentation électrique (380b), un bloc-batterie (308a) ou une soufflante (308d).

4. Système d'assainissement portable (100) selon l'une quelconque des revendications 1-3, dans lequel les un ou plusieurs modules composants incluent l'ensemble perche (102).

5. Système d'assainissement portable (100) selon la revendication 1-3, dans lequel le contenant comprend en outre un ou plusieurs récipients au sein de la chambre interne (178), et dans lequel les un ou plusieurs modules composants sont configurés pour être fixés de manière amovible aux un ou plusieurs récipients par le biais d'une ou plusieurs interfaces de couplage.

6. Système d'assainissement portable (100) selon la revendication 5, dans lequel les une ou plusieurs interfaces de couplage comprennent une fiche et une prise, un ou plusieurs dispositifs de verrouillage, un ou plusieurs crans, ou un ou plusieurs boutons-pression.

7. Système d'assainissement portable (100) selon l'une quelconque des revendications 1-3, dans lequel le contenant comprend en outre une étiquette d'identification par radiofréquence (RFID).

8. Système d'assainissement portable (100) selon l'une quelconque des revendications 1-3, comprenant en outre un cadre de retenue fixé au contenant, dans lequel le cadre de retenue est configuré pour loger un ou plusieurs modules composants supplémentaires.

9. Système d'assainissement portable (100) selon l'une quelconque des revendications 1-3, dans lequel le contenant comprend en outre une fenêtre (390) qui est configurée pour permettre de visualiser au moins une partie de la chambre interne (178).

10. Système d'assainissement portable (100) selon l'une quelconque des revendications 1-3, dans lequel les un ou plusieurs modules composants comprennent une alimentation électrique (308b), dans lequel l'alimentation électrique (308b) comprend un commutateur d'activation (392) et un écran d'informations (394), et dans lequel des indicateurs d'état sont affichés sur l'écran d'informations (394).

11. Système d'assainissement portable (100) selon l'une quelconque des revendications 1-10, dans lequel le contenant comprend en outre un dispositif à système de positionnement mondial (GPS).

12. Système d'assainissement portable (100) selon la revendication 3, dans lequel le microcontrôleur (398) est configuré pour détecter une marque et un modèle des une ou plusieurs alimentations électriques (308b) fixées, du bloc-batterie (308a) et/ou de la soufflante (308d).

13. Système d'assainissement portable (100) selon l'une quelconque des revendications 1-3, comprenant en outre un flexible (122) qui relie l'ensemble perche (102) au contenant, dans lequel le flexible (122) comprend un premier raccord qui se raccorde de manière amovible à un second raccord du contenant.

14. Système d'assainissement portable (100), comprenant :
un contenant comprenant :
une chambre interne (178) ;
une fenêtre (390) qui est configurée pour permettre de visualiser au moins une partie de la chambre interne (178) ;
un microcontrôleur (398) ;
un premier raccord ; et
des modules composants fixés de manière amovible à l'intérieur de la chambre interne (178), dans lequel le microcontrôleur (398) est configuré pour détecter, sur la base d'un agencement de broches de connexion, la marque et le modèle des un ou plusieurs modules composants, dans lequel le microcontrôleur (398) compare les données d'agencement de broches de connexion installées d'un ou plusieurs modules composants à des agencements connus stockés dans une mémoire pour déterminer la compatibilité des un ou plusieurs modules composants et dans lequel les modules composants comprennent :
un ensemble perche (102) comprenant une tête d'assainissement (106) présentant une lampe à ultraviolets (UV) (140) ;
une alimentation électrique (380b) incluant un commutateur d'activation et un écran d'informations (394), dans lequel des indicateurs d'état sont affichés sur l'écran d'informations (394) ;
un bloc-batterie (308a) ; et
une soufflante (308d) ; et
un flexible (122) qui raccorde l'ensemble perche (102) au contenant, dans lequel le flexible (122) comprend un second raccord qui se raccorde de manière amovible au premier raccord du contenant.
